# EUROPEAN PATENT SPECIFICATION

(11) **EP 1 699 840 B1**
(45) Date of publication and mention of the grant of the patent: **22.01.2014**
(21) Application number: 04815527.9
(22) Date of filing: 22.12.2004
(51) Int. Cl.: C08G 18/18, C08G 18/76, C08G 18/48, C07C 249/02

(54) **NON-FUGITIVE CATALYSTS CONTAINING IMINE LINKAGES AND TERTIARY AMINES, AND POLYURETHANE PRODUCTS MADE THEREFROM**
NICHTFLÜCHTIGE KATALYSATOREN MIT IMINBINDUNGEN UND TERTIÄREN AMINEN UND DARAUS HERGESTELLTE POLYURETHANPRODUKTE
CATALYSEURS NON FUGITIFS CONTENANT DES LIAISONS IMINE ET DES AMINES TERTIAIRES, ET PRODUITS EN POLYURETHANNE OBTENUS A PARTIR DE CEUX-CI

(30) Priority: 23.12.2003 US 531935 P
(43) Date of publication of application: 13.09.2006
(73) Proprietor: Dow Global Technologies LLC, Midland, MI 48674 (US)
(72) Inventor: DRUMRIGHT, Ray, E., Midland, MI 48640 (US); PRANGE, Robbyn, Pearland, TX 77584 (US); CASATI, François, M., CH-8808 Pfaffikon (CH)
(74) Representative: Raynor, John
(86) International application number: PCT/US2004/043462
(87) International publication number: WO 2005/063840

(56) References cited:
- RU-C1- 2 134 259
- RU-C2- 2 161 603
- RU-C2- 2 163 608
- US-A- 3 050 475
- US-A1- 2003 187 155
- M. K. SAHA ET AL.: "Structure and Magnetic Properties of an Oxalic Acid Bridged Dinuclear Copper(II) Complex" ZEITSCHRIFT FÜR NATURFORSCHUNG B, vol. 53, 1998, pages 1281-1284, XP009044977

## Description

The present invention pertains to non-fugitive catalysts containing an imine linkage and a tertiary amine, and polyurethane polymer products produced with such catalysts.

Polyether polyols based on the polymerization of alkylene oxides, and/or polyester polyols, are the major components of a polyurethane system together with isocyanates. These systems generally contain additional components such as cross-linkers, chain extenders, surfactants, cell regulators, stabilizers, antioxidants, flame retardant additives, eventually fillers, and typically catalysts such as tertiary amines and/or organometallic salts.

Organometallic catalysts, such as lead or mercury salts, can raise environmental issues due to leaching upon aging of the polyurethane products. Others, such as tin salts, are often detrimental to polyurethane aging.

The commonly used tertiary amine catalysts, give,rise to several problems, particularly in flexible, semi-rigid and rigid foam applications. Freshly prepared foams using these catalysts often exhibit the typical odor of the amines and give rise to increased fogging (emission of volatile products).

The presence, or formation, of tertiary amine catalyst vapors in polyurethane products having vinyl films or polycarbonate or polyester/polyether elastomer, such as Hytrel* thermoplastic polyester elastomer (Trademark of DuPont) sheets exposed thereto can be disadvantageous. Such products commonly appear in automotive as well as in many domestic applications. Specifically, the tertiary amine catalysts present in polyurethane foams have been linked to the staining of the vinyl film and degradation of polycarbonate or Hytrel sheets. This PVC staining and polycarbonate or Hytrel decomposition problems are especially prevalent in environments wherein elevated temperatures exist for long periods of time, such as in automobile interiors when left in the sunlight.

Various solutions to the above problems have been proposed. One is the use of amine catalysts which contain an isocyanate reactive group, i.e. a hydroxyl or a primary and/or a secondary amine. Such a compound is disclosed in EP Publication 747,407. Other types of reactive monol catalysts are described in U.S. Patents 4,122,038, 4,368,278 and 4,510,269. Since the monols are monofunctional, these reactive amines act as chain stoppers and have a detrimental effect on the polymer build up and affect polyurethane product physical characteristics.

Use of specific amine-initiated polyols is proposed in EP 539,819, in U.S. Patent 5,672,636 and in WO 01/ 58,976.

Various other publications have reported polyols having autocatalytic activity and can replace all or a portion of conventional amine catalysts. See for example U.S. Patent 5,672,636; European Patent Publications 0 047 371, 1 268 598 and 1 319 034; and WO Publications 03/016372, 03/029320 and 03/055930.

Capping of conventional polyether polyols with N,N-dialkylglycidylamine is claimed in US 3,428,708. While this process gives polyols with autocatalytic activity, it is restricted to dialkylamino groups which are mainly active to catalyze the water-isocyanate reaction and much less the polyolisocyanate reaction.

U.S. Patent 3,050,475 describes production of polyurethane plastics using Schiff's base catalysts.

RU 2 163 608 describes a catalyst for hydroxyl-containing component and a hydroxyl-containing component for production of elastic polyurethane foam.

Zeitschrift Fur Naturforschung B, vol, 53, 1998, pages 1281-1284 discusses structure and magnetic properties of an oxalic acid bridged dinuclear copper (II) complex. This document discloses synthesis of [LCu{u-(OH)2(C2O2)}CuL](ClO4)2, (HL = N,N-dimethyl N-propylsalicylald-imine).

U.S. Patent Application 2003/0187155 describes epoxy hardeners for low temperature curing.

RU 2 134 259 describes N,N-dimethylaminomethylated aromatic Schiff bases as hardening accelerators of epoxy resins, and method of preparing thereof.

RU 2 161 603 describes Bis-N,N-dimethylaminomethylated aromatic Schiff bases and methods of preparation thereof.

Despite the advances made in the art, there continues to be a need for improved catalysts for producing polyurethane products and/or catalysts which can reduce or eliminate the amount of fugitive amine catalysts and/or organometallic salts used in producing polyurethanes.

It is also desirable to have an industrial process to manufacture polyols having autocatalytic properties where the autocatalytic polyols do not interfere with conventional polyol production or polyurethane product processes and characteristics.

It is an object of the present invention to produce polyurethane products containing a reduced level of conventional tertiary amine catalysts, a reduced level of reactive amine catalysts or polyurethane products produced without the need of such amine catalysts. It is ananother objective of the present invention to produce polyurethane products containing a reduced level of organometallic catalyst or to produce such products in the absence of organometallic catalysts.

It is another object of the invention to have a process to adjust the manufacturing conditions, or reactivity, of polyurethane products by using non-fugitive catalysts of the present invention.

It is a further object of the present invention to increase productivity by combining non-fugitive catalysts with conventional catalysts to obtain faster processes in the manufacture of polyurethane products.

It is a further object of the present invention to provide non-fugitive catalysts possessing an imine linkage and a tertiary amine so the industrial manufacturing process of the polyurethane product using these compounds and the physical characteristics of the polyurethane products made therefrom are not adversely affected and may even be improved by the reduction in the amount of conventional or reactive amine catalysts or in elimination of the amine catalyst, and/or by reduction or elimination of organometallic catalysts.

The present invention is a catalyst composition wherein the catalyst has at least one imine linkage and at least one tertiary amine group.
The present invention is a catalyst composition wherein the catalyst has at least one imine linkage and at least one tertiary amine moiety wherein the imine linkage is obtained by the reaction mixture comprising
(i) a compound having at least one aldehyde or ketone moiety and
(ii) a compound having at least one primary amine moiety and at least one tertiary amine moiety
wherein (i) is mixture of (A) a compound containing at least one epoxy moiety with (B) a compound containing an epoxy reactive moiety and an aldehyde or ketone.

In another embodiment, the present invention is a polyol composition containing from 99.9 to 50 percent by weight of a polyol compound having a functionality of 2 to 8 and a hydroxyl number of from 20 to 800 and from 0.1 to 50 percent of a catalyst composition wherein the catalyst has at least one imine linkage and at least one tertiary amine group wherein the imine linkage is obtained by the reaction mixture comprising
(i) a compound having at least one aldehyde or ketone moiety and
(ii) a compound having at least one primary amine moiety and at least one tertiary amine moiety
wherein (i) is mixture of (A) a compound containing at least one epoxy moiety with (B) a compound containing an epoxy reactive moiety and an aldehyde or ketone. Preferably the amount of catalyst is present from 0.5 to 10 parts by weight of the polyol.

In a further embodiment, the present invention is a process for the production of a polyurethane product by reaction of a mixture of
(a) at least one organic polyisocyanate with
(b) a polyol composition with the polyols having a calculated nominal functionality between 2 to 8 and a hydroxyl number of from 20 to 800 mg KOH/g and
(c) at least one non-fugitive catalyst containing at least one imine linkage and at least one tertiary amine group wherein the imine linkage is obtained by the reaction mixture comprising
   (i) a compound having at least one aldehyde or ketone moiety and
   (ii) a compound having at least one primary amine moiety and at least one tertiary amine moiety
      wherein (i) is mixture of (A) a compound containing at least one epoxy moiety with (B) a compound containing an epoxy reactive moiety and an aldehyde or ketone
(d) optionally in the presence of another catalyst and/or blowing agent; and
(e) optionally additives or auxiliary agents known per se for the production of polyurethane foams, elastomers or coatings.

In another embodiment, the present invention is a process as disclosed above wherein catalyst (c) contains at least one isocyanate reactive hydrogen.

In another embodiment, the catalyst (c) is a gelling catalyst, i.e. catalyzes the reaction between the polyol and isocyanate.

In another embodiment, the catalyst (c) is a liquid polymer with a molecular weight above 500.

In another embodiment, the catalyst (c) contains more than one catalytically active tertiary amine moiety.

In another embodiment, the catalyst (c) contains some aldehyde and/or ketone moieties.

In another embodiment, the catalyst (c) is stable against hydrolysis at room temperature.

In another embodiment, the catalyst (c) is combined with a polyol having autocatalytic properties when producing a polyurethane product.

In another embodiment, the present invention is a process as disclosed above wherein catalyst (c) contains at least one isocyanate reactive hydrogen

In another embodiment, the present invention is a process as disclosed above wherein catalyst (c) contains at least one isocyanate reactive hydrogen and the polyisocyanate (a) contains at least one polyisocyanate that is a reaction product of an excess of polyisocyanate with catalyst (c).

In a further embodiment, the present invention is a process as disclosed above where catalyst (c) contains at least one isocyanate reactive hydrogen and the polyol (b) contains a prepolymer obtained by the reaction of an excess of catalyst (c) with a polyisocyanate.

The invention further provides for polyurethane products produced by any of the above processes.

Non-fugitive catalysts (c) accelerate the addition reaction of organic polyisocyanates with polyhydroxyl or polyamino compounds and the reaction between the isocyanate and the blowing agent such as water or a carboxylic acid or its salts. The addition of these catalysts (c) to a polyurethane reaction mixture reduces or eliminates the need to include a conventional tertiary amine catalyst within the mixture or an organometallic catalyst. In combination with conventional amine catalysts and/or autocatalytic polyols, the present catalysts (c) can also reduce the mold dwell time in the production of molded foams or improve some polyurethane product properties.

The use of such catalysts (c) reduces the need for conventional fugitive amine catalysts and the associated disadvantages of vinyl staining or degradation of polycarbonate of Hytrel elastomer sheets. The advantages of the present catalysts (c) are achieved by including in the reaction mixture for polyurethane products either non-fugitive catalysts (c) containing imine linkages and tertiary amines, or by including such catalysts (c) containing reactive hydrogens as feedstock in the preparation of SAN, PIPA or PHD copolymer polyols or adding them to the polyurethane reaction mixture or by using such catalysts (c) in a prepolymer with a polyisocyanate alone or with an isocyanate and a second polyol.

As used herein the term polyols are those materials having at least one group containing an active hydrogen atom capable of undergoing reaction with an isocyanate. Preferred among such compounds are materials having at least two hydroxyls, primary or secondary, or at least two amines, primary or secondary, carboxylic acid, or thiol groups per molecule. Compounds having at least two hydroxyl groups or at least two amine groups per molecule are especially preferred due to their desirable reactivity with polyisocyanates.

Suitable polyols that can be used to produce polyurethane materials with the non-fugitive catalysts (c) of the present invention are well known in the art and include those described herein and any other commercially available polyol and/or SAN, PIPA or PHD copolymer polyols. Such polyols are described in "Polyurethane Handbook", by G. Oertel, Hanser publishers. Mixtures of one or more polyols and/or one or more copolymer polyols may also be used to produce polyurethane products according to the present invention.

Representative polyols include polyether polyols, polyester polyols, polyhydroxy-terminated acetal resins, hydroxyl-terminated amines and polyamines. Examples of these and other suitable isocyanate-reactive materials are described more fully in U.S. Patent 4,394,491. Alternative polyols that may be used include polyalkylene carbonate-based polyols and polyphosphate-based polyols. Preferred are polyols prepared by adding an alkylene oxide, such as ethylene oxide, propylene oxide, butylene oxide or a combination thereof, to an initiator having from 2 to 8, preferably 2 to 6 active hydrogen atoms. Catalysis for this polymerization can be either anionic or cationic, with catalysts such as KOH, CsOH, boron trifluoride, or a double metal cyanide complex (DMC) catalyst such as zinc hexacyanocobaltate or quaternary phosphazenium compound. Their unsaturation is between 0.001 and 0.1 meq/g. After production, the catalyst is removed when it is alkaline. The polyol may be neutralized by the addition of an inorganic or organic acid, such as a carboxylic acid or hydroxyl-carboxylic acid.

The polyol or blends thereof employed depends upon the end use of the polyurethane product to be produced. The molecular weight or hydroxyl number of the base polyol may thus be selected so as to result in flexible, semi-flexible, integral-skin or rigid foams, elastomers or coatings, or adhesives when the polymer/polyol produced from the base polyol is converted to a polyurethane product by reaction with an isocyanate, and depending on the end product in the presence of a blowing agent. The hydroxyl number and molecular weight of the polyol or polyols employed can vary accordingly over a wide range. In general, the hydroxyl number of the polyols employed may range from 20 to 800. Selection of a polyol with the appropriate hydroxyl number, level of ethylene oxide, propylene oxide and butylene oxide, functionality and equivalent weight are standard procedures known to those skilled in the art. For example, polyols with a high level of ethylene oxide will be hydrophilic, while polyols with a high amount of propylene oxide or butylene oxide will be more hydrophobic.

In the production of a flexible polyurethane foam, the polyol is preferably a polyether polyol and/or a polyester polyol. The polyol generally has an average functionality ranging from 2 to 5, preferably 2 to 4', and an average hydroxyl number ranging from 20 to 100 mg KOH/g, preferably from 20 to 70 mgKOH/g. As a further refinement, the specific foam application will likewise influence the choice of base polyol. As an example, for molded foam, the hydroxyl number of the base polyol may be on the order of 20 to 60 with ethylene oxide (EO) capping, and for slabstock foams the hydroxyl number may be on the order of 25 to 75 and is either mixed feed EO/PO (propylene oxide) or is only slightly capped with EO or is 100 percent PO based. For elastomer applications, it will generally be desirable to utilize relatively high molecular weight base polyols, from 2,000 to 8,000, having relatively low hydroxyl numbers, for example, 20 to 50.

For the production of visco-elastic foams, i.e. flexible foams with very low resiliency, a combination of polyols with different hydroxyl numbers, up to 300, and functionalities between 1 and 4, is used.

Typically polyols suitable for preparing rigid polyurethanes include those having an average molecular weight of 100 to 10,000 and preferably 200 to 7,000. Such polyols also advantageously have a functionality of at least 2, preferably 3, and up to 8, preferably up to 6, active hydrogen atoms per molecule. The polyols used for rigid foams generally have a hydroxyl number of 200 to 1,200 and more preferably from 300 to 800.

For the production of semi-rigid foams, it is preferred to use a trifunctional polyol with a hydroxyl number of 30 to 80.

The initiators for the production of polyols generally have 2 to 8 functional groups that will react with the alkylene oxide. Examples of suitable initiator molecules are water, organic dicarboxylic acids, such as succinic acid, adipic acid, phthalic acid and terephthalic acid and polyhydric, in particular dihydric to octahydric alcohols or dialkylene glycols, for example ethanediol, 1,2- and 1,3-propanediol, diethylene glycol, dipropylene glycol, 1,4-butanediol, 1,6-hexanediol, glycerol, trimethylolpropane, pentaerythritol, sorbitol and sucrose or blends thereof. Other initiators include linear and cyclic amine compounds such as ethanolamine, triethanolamine, and various isomers of toluene diamine.

Polyols having autocatalytic activity may also be used as the polyol or in combination with the polyols described above. In general such autocatalytic polyols contain an easily accessible tertiary amine moiety. Description of such autocatalytic polyols can be found in U.S. Patent 5,672,636; European Patent Publications 0 047 371, 1 268 598 and 1 319 034; and WO Publications 03/016372, 03/029320 and 03/055930.

Structure properties of the autocatalytic polyols in relation to polyurethane product end use is generally the same as for polyols described above. Generally the tertiary amine of such autocatalytic polyols can be part of the initiator, part of the polyol chain, and/or part of the polyol end capping. These tertiary amine groups give autocatalytic characteristics to such polyols.

The limitations described with respect to the characteristics of the polyols above are not intended to be restrictive but are merely illustrative of the large number of possible combinations for the polyol or polyols used.

Non-fugitive catalysts (c) containing at least one imine linkage and one tertiary amine group are based on the reaction between an aldehyde, or a ketone, and a molecule containing both primary amine and tertiary amine groups. The non-fugitive aspect of catalyst (c) is believed to be due to either through its bulky molecular mass which is at least 150 g/mol or through its isocyanate reactive moieties, or through both features. Alternatively the imino group can react with isocyanate during the polyurethane product reactions as described in EP 363,008 albeit without catalytic effect in this latter document. A further advantage of the non-fugitive catalysts (c) is the imine bond formed is stable to hydrolysis at room temperature.

Several chemistries are possible to obtain non-fugitive catalysts (c) as it will be explained hereafter under (c1), (c2), (c3), (c4), (c5), (c6) (c7), (c8) or (c9).

Catalysts (c1) are obtained by reacting a molecule containing either at least one aldehyde or one ketone group with a primary amine moiety of a molecule containing a primary amine and at least one tertiary amine group. The final compound has a molecular weight higher than 150. The ketones and aldehydes for use in the present invention are as generally known in the art defined by R-C(O)-R¹ and R-C(O)-H, respectively where R and R¹ are moieties which do not react with the primary amine under conditions necessary to form an imine. Typically R and R¹ are independently a C1-C20, preferable C1-C15, substituted or unsubstituted linear or branched alkyl, a cyclic, heterocyclic or aromatic compounds containing 4 to 20 atoms, preferably 5 to 15 atoms in the ring, or R and R¹ may be bound to each other to form a ring structure containing 5 to 20 atoms in the ring. The ring structures may be further substituted. The variation of substituted ring structures is exemplified by the compounds listed herein. Non limiting substitutes include hydroxyl, amines, carboxylic acids, alkyl or alkyl oxide moieties. The term ring structure as used herein includes compounds which contain more than one ring, such as naphtalene for an aromatic ring structure.

Examples of aldehydes are salicylaldehyde, vannilin, 3-hydroxybenzaldehyde, 4-hydroxybenzaldehyde, 4-dimethylaminobenzaldehyde, benzaldehyde, furfural, anisaldehyde, tolualdehyde, isophthalaldehyde, phthalic dicarboxyaldehyde, terephthaldicarboxaldehyde, 4-(dimethylamino)benzaldehyde, 4-(diethylamino)benzaldehyde, 4-(dibutylamino)benzaldehyde, 4-[3-(dimethylamino)propoxylbenzaldehyde, nitrobenzaldehyde, chlorobenzaldehyde, 2-carboxybenzaldehyde, phenyl-1,3-dicarboxyaldehyde, dihydroxybenzaldehydes, trihydroxybenzaldehydes, piperonal, beta-hydroxybutyric aldehyde (aldol), omega-hydroxymethylfurfural, hydroxy-acetaldehyde, 5-hydroxy-pentanal, acetaldol, 2,5-dimethyl-2-hydroxy adipaldehyde, 3-(beta-hydroxyethoxy)-propanal, beta-hydroxyacetaldehyde. Prefered compounds are aromatic based aldehydes such as salicylaldehyde, 4-dimethylaminobenzaldehyde, 4-hydroxybenzaldehyde or vannilin.

Examples of ketones are cyclohexanone, methylcyclohexanone, cyclopentanone, methylisobutylketone, tropolone, tropone, 2'-hydroxyacetophenone, 4'-hydroxyacetophenone, 3'-hydroxyacetophenone, 3-acetyl-1-propanol, 4-hydroxy-3-methyl-2-butanone, 4-hydroxy-4-methyl-2-pentanone, 4'-hydroxyvalerophenone, dihydroxyacetophenone, benzyl-4-hydroxyphenylketone, acetovanillone, aminobenzophenone, aminobenzoquinone.

Examples of amines bearing both primary and tertiary amine groups are 3-(dimethylamino)-propylamine, 1-(3-aminopropyl)-imidazole, 1-(3-aminopropyl)-2-methylimidazole, N,N-dimethyldipropylenetriamine, N,N-dimethylethylene diamine, N,N-diethylethylene diamine, N,N-dibutylethylene diamine, 3-(diethylamino)-propylamine, 3-(dibutylamino)-propylamine, N,N,2,2-tetramethyl-1,3-propanediame, 2-amino-5-diethylaminopentane, N-methyl- (N'-aminoethyl)-piperazine, 1,4-bis(3-aminopropyl)piperazine, 3-aminoquinuclidine, 4'-(2-aminoethyl)morpholine, 4-(3-aminopropyl)morpholine, N,N-dimethyl-1,4-phenylenediamine, 5-amino-1-ethylpyrazole, 2-aminopyridine, 2-(aminomethyl)pyridine, 2-(aminoethyl)pyridine, 4-aminopyridine, 3-aminopyridine, 3-(aminomethyl)pyridine, N-aminopropyl pyrrolidine 2-aminopicolines, diaminopyridines, 2-aminopyrimidine, 4-aminopyrimidine, aminopyrazine, 3-amino-1,2,4-triazine, aminoquinolines, N,N dimethyldipropylenetriamine and 3,3'-diamino-N-methyl dipropylamine, N-methyl-1,3-propyldiamine.

Catalysts (c2) are obtained by reacting a molecule, containing at least one aldehyde or one ketone group and at least one tertiary amine, with a molecule containing a primary amine and optionally other amine and/or alcohol moieties.

The ketones and aldehydes containing a tertiary amine can be generally represented by the (R²)₂N-R³-C(O)-R and (R²)₂N-R³-C(O)H where R is as defined above, R² is a C1-C6 linear or branched alkyl and R³ is a C1 to C12 linear or branched alkyl, an aromatic or alkyl aromatic moiety having 6 to 20, preferably 6 to 15 carbon atoms substituted with at least one tertiary amine or R³ and R may be bound to each other to form a ring structure having 5 to 20 atoms, preferably 5 to 15 atoms in the ring. R³ may also be a cyclic or bicyclic moiety having 5 to 20 atoms wherein at least one nitrogen is included in the ring structure The alkyl and ring moieties may be substituted with various moieties as described above.

Examples of aldehydes and ketones containing a tertiary nitrogen are quinuclidinone, tropinone, 1-methyl-4-piperidinone, 4-(dimethylamino)benzaldehyde, 4-(diethylamino)benzaldehyde, 4-(dibutylamino)benzaldehyde, 4- [3-(dimethylamino)propoxy]benzaldehyde.

Compounds containing primary amines are well known in the art. Representative examples of preferred compounds containing one or more primary amines are ethylenediamine, 1,6-hexanediamine, aniline, N,N-dimethyldipropylenetriamine, 3,3'-diamino-N-methyl-dipropylamine,3-aminopropyl-N-methylethanolamine and 3-(dimethylamino)propylamine, monoethanolamine, 2-amino-1-butanol.

Catalyst (c3) are catalysts obtained by modification of epoxy functional molecules with compounds bearing both an aldehyde or ketone and an epoxy reactive moiety such as an alcohol, an amine, a thiol or a carboxylic acid, followed by subsequent reaction with a primary amine molecule bearing tertiary amine to form an imine linkage. Catalysts (c3) preferably contain more than one imine linkage and more than one tertiary amine group per molecule.

For the present invention, compounds having aldehyde functionality and epoxide reactive functionality (alcohol, amine, thiol or carboxylic acid) are C3-C30, preferably C5-C18, aliphatic, aromatic or polyaromatic compounds and ring structures containing a heteroatom, where the aldehyde moiety is attached directly to the ring and the epoxide reactive moiety is bonded directly to the ring or via a C1 to C6, linear or branched, alkyl moiety. Such compounds may contain more than one epoxide reactive moiety or more than one aldehyde moiety. The ring constitutes may be further substituted with groups that do not react with an epoxy, such as an alkyl or alkoxy moiety.

Examples of alcohols bearing aldehyde functionality are salicylaldehyde, vanillin, 5-(hydroxymethyl)-furfural, 3-hydroxybenzaldehyde, 4-hydroxybenzaldehyde, dihydroxybenzaldehydes, and trihydroxybenzaldehydes.

Examples of carboxylic acids bearing aldehyde functionality are 2-carboxybenzaldehyde and 3-carboxybenzaldehyde.

For the present invention, compounds having ketone functionality and epoxide reactive functionality (alcohol, amine, thiol or carboxylic acid) are C3-C30, preferably C5-C18, aliphatic, aromatic or polyaromatic compounds and ring structures containing a heteroatom, where the epoxide reactive moiety is bonded directly to the ring or via a C1 to C6, linear or branched, alkyl moiety. The ketone may also be part of the ring structure. Such compounds may contain more than one epoxide reactive moiety or more than one ketone moiety. The ring constitutes may be further substituted with groups that do not react with an epoxy, such as an alkyl or alkoxy moiety.

Examples of alcohols bearing ketone functionality are 2'-hydroxyacetophenone, 4'-hydroxyacetophenone, 3'-hydroxyacetophenone, 3-acetyl-1-propanol, 4-hydroxy-3-methyl-2-butanone, 4-hydroxy-4-methyl-2-pentanone, 4'- hydroxyvalerophenone, dihydroxyacetophenone, benzyl-4-hydroxyphenylketone and acetovanillone.

Examples of amine bearing ketones are 3'-aminoacetophenone, 4'-aminoacetophenone, and aminobenzophenone. Examples of carboxylic acid containing ketones are 4-acetylbenzoic acid and 2-benzoylbenzoic acid.

Examples of epoxides, or epoxy resins, for producing the catalysts (c3) are known in the art. See for example, U.S. Patent 4,609,685. The epoxide materials can be monomeric or polymeric, saturated or unsaturated, aliphatic, cycloaliphatic, aromatic or heterocyclic and may be substituted if desired with other substituents besides the epoxy groups, e.g., hydroxyl, groups, ether radicals and halogen atoms. A preferred family of polyepoxides can be represented by the formula 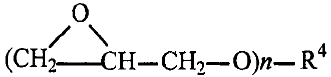 wherein R⁴ is substituted or unsubstituted aromatic, aliphatic, cycloaliphatic or heterocyclic group and n has an average value of from 1 to 8.

Examples of preferred epoxides are phenyl glycidyl ether, aromatic epoxy resins of bis-phenol A, bisphenol F, and resorcinol, and hydrogenated versions thereof; and aliphatic polyether based epoxies such as D.E.R. 736, D.E.R. 732 and ERL-4221 (cyclic aliphatic epoxide) all available from The Dow Chemical Company. Other preferred epoxies include epoxidized oils such as epoxidized soybean oil and epoxidized linseed oil. A mixture of any two or more expoxides can be used in the practice of the present invention. Preferably the epoxide resin has an average equivalent weight of 90 to 1000. More preferably the epoxy resin has an average equivalent weight of 150 to 500.

Preferred epoxides are aliphatic or cycloaliphatic polyepoxides, more preferably diepoxides such as D.E.R. 732 or D.E.R. 736 or low chlorine epoxy resins with similar structures.

Example of amines bearing both a primary and a tertiary amine groups are described above under section (c1).

Catalysts (c4) are produced analogously to catalysts (c3) with the exception that part of the aldehyde or ketone bearing epoxide reactive functionality that is reacted with polyepoxide is replaced by a reagent bearing only epoxide reactive functionality. This substitution allows for the average molecular weight of the final catalyst (c4) to be adjusted up, by chain-extending the poly-epoxide using polyfunctional compounds, or down by chain-stopping the poly-epoxide using monofunctional compounds, to tailor the product for a specific application. Examples of molecules suitable for substitution of a fraction of the aldehyde or ketone bearing epoxy reactive functionality include phenol, cresol, bis phenol A, bisphenol F, novolak polyols, resorcinol, ethylenediamine, 3,3'-diamino-N-methyl-dipropylamine, monethanolamine, acetic acid, adipic acid, succinic acid, isophthalic acid, phthalic acid, and terephthalic acid.

Catalysts (c5) are produced analogously to catalysts (c3) with the exception that some of the primary amine substituted with tertiary amine is replaced by a multifunctional primary amine. This substitution allows for the average molecular weight of the final catalyst (c5) to be adjusted up or down to tailor the product for a specific application. Examples of molecules suitable for this substitution include monoethanolamine, 2-amino-1-butanol, 2-amino-2-ethyl-1,3-propanediol, ethylene diamine, butane diamine, hexane diamine, JEFFAMINE® polyoxyalkyleneamines (trademark of Huntsman Chemical Corporation), methylenedianiline, and diaminobenzene.

Another option to produce non fugitive catalyst with multiple active sites is (c6), based on the reaction of polyols capped with primary amines, such as JEFFAMINE polyoxyalkyleneamine, with a molecule containing an aldehyde or a ketone group and a tertiary amine, such as those described under (c2). The general structures of the JEFFAMINE polyoxyalkyleneamines are known, as per Huntsman's technical bulletin 1008-1002.

Catalysts (c7) are identical to (c3) and/or (c4) but part of the epoxy resin has been reacted with a compound containing an epoxide reactive moiety, such as an amine, prior to addition of the aldehyde or ketone bearing epoxide reactive functionality. For example, the epoxy is reacted with a secondary amine bearing tertiary amine functionality (like imidazole) or a primary amine such as monoethanolamine, or aniline which allows for adjustment of the functionality and molecular weight of the final product. Chain extension compounds are those listed under (c3). Preferably.the compound that is pre-reacted with the poly-epoxide contains also a tertiary amine moiety. Generally 1 to 50 percent of the epoxy groups will be reacted out in this step.

Generally, secondary amines can be represented by HNR₂⁵ and primary amines by H₂NR⁵ where each R⁵ is independently a compound having 1 to 20 carbon atoms or may be attached together with the nitrogen atom and optionally other hetero atoms and alkyl-substituted hetero atoms to form a saturated heterocyclic ring.

Examples of epoxy reactive amines that are commercially available and that can be used to manufacture catalyst (c7) are methylamine, dimethylamine, diethylamine, N,N-dimethylethanolamine, N,N'-dimethylethylenediamine, N,N-dimethyl-N'-ethylenediamine, 3-dimethylamino-1-propanol, 1-dimethylamino-2-propanol, 3-(dimethylamino) propylamine, dicyclohexylamine, 4,6-dihydroxypyrimidine, 1-(3-aminopropyl)-imidazole, 3-hydroxymethyl quinuclidine, 2-methyl imidazole, 1-(2-aminoethyl)-piperazine, 1-methyl-piperazine, 3-quinuclidinol, 2,4-diamino-6-hydroxypyrimidine, 2,4-diamino-6-methyl-1,3,5-triazine, 3-aminopyridine, 2,4-diaminopyrimidine, 2-phenyl-imino-3-(2-hydroxyethyl)-oxazalodine,N-(-2-hydroxyethyl)-2-methyl-tetrahydropyrimidine, N-(2-hydroxyethyl)-imidazoline,2,4-bis-(N-methyl-2-hydroxytethylamino)-6-phenyl-1,3,5-triazine, bis-(dimethylaminopropyl)amino-2-propanol, tetramethylamino-bis-propylamine, 2-(2-aminoethoxy)-ethanol, N,N-dimethylaminoethyl-N'-methyl ethanolamine, 2-(methylamino)-ethanol, 2-(2-methylaminoethyl)-pyridine, 2-(methylamino)-pyridine, 2-methylaminomethyl-1,3-dioxane, dimethylaminopropyl urea.

Compounds containing at least one tertiary nitrogen and at least one hydrogen molecule reactive to an epoxide can be represented by ((H)ₓ-A-R⁶)z-M-(R⁷)y where A is nitrogen or oxygen; x is 2 when A is nitrogen and 1 when A is oxygen, R⁶ and R⁷ are linear or branched alkyl groups having 1 to 20 carbon atoms; M is an amine or polyamine, linear or cyclic with at least one tertiary amine group; y is an integer from 0 to 6; and z is an integer from 1 to 6.

Compounds containing both a tertiary nitrogen and a primary amine can be represented by the formula: H₂N - R⁸- N(R⁹)₂ where R³ is an aliphatic or cyclic chain having 1 to 20 carbon atoms and R⁹ is a C1 to C3 alkyl group.

Catalyst (c8) is obtained by reaction of an isocyanate with an alcohol bearing aldehyde or ketone functionalities, followed by subsequent reaction with a primary amine bearing tertiary amine to form imine linkage to the polyol.

Examples of isocyanates are toluene-diisocyanate, isophorone-diisocyanate, phenylisocyanate, methyldiphenyl-isocyanate, blends or prepolymers thereof. Preferred isocyanates are polyisocyanates, more preferably diisocyanates.

Examples of alcohols bearing aldehyde ketone functionality and amines bearing both a primary and a tertiary amine are described above.

Catalyst (c9) is based on the combination of chemistries described under (c3) and (c8), i.e. by mixing epoxy and isocyanate based catalysts.

The starting materials for the Production of catalyst (c) are commercially available or can be made by procedures known to those skilled in the art, as are the reaction conditions for producing the catalyst (c). In general the ratio of compounds in a particular reaction step is such that there is close to a molar stoichiometric equivalent of reactive moieties, that is from 0.9:1, preferably from 0.95: 1 to 1:1. For example, in the production of catalyst (c3), when the reaction is between the functionalized epoxy, such as an aldehyde moiety, and primary amine, the molar equivalents of aldehyde to primary amine is approximately 1:1. However, it can be increased to 1.2:1 when one wants to minimize the amount of free amine in catalyst (c). With catalysts (c4) and (c5) one may adjust this ratio to have a molar excess of one of the reactive groups for increasing the molecular weight.

The weight ratio of non-fugitive catalyst (c) in relation to polyol will vary depending on the amount of additional catalyst one may desire to add to the reaction mix and to the reaction profile required by the specific application. Generally if a reaction mixture with a base level of catalyst having specified curing time, non-fugitive catalyst (c) is added in an amount so that the curing time is equivalent where the reaction mix contains at least 10 percent by weight less catalyst. Preferably the addition of (c) is added to give a reaction mixture containing 20 percent less catalyst than the base level. More preferably the addition of (c) will reduce the amount of catalyst required by 30 percent over the base level. For some applications, the most preferred level of (c) addition is where the need for a volatile tertiary or reactive amine catalysts or organometallic salt is eliminated. In some other applications, such as for decreasing the demold time, it is desirable to maintain the standard amount of conventional amine or organometallic catalyst and add the present catalyst (c) in an amount to enhance the demold time. For this later application, generally 0.1 to 10 parts or greater of catalyst (c) are added per 100 parts by weight of polyol.

The adjustment of the level of catalyst (c), to be used alone or in combination with conventional polyurethane catalysts or polyols containing autocatalytic activity, for a particular application is well known to those skilled in the art.

Combination of two or more non-fugitive catalysts of (c) type can also be used with satisfactory results in a single polyurethane formulation when one wants for instance to adjust blowing and gelling reactions modifying the catalysts (c) structure with different tertiary amines, functionalities, equivalent weights, EO/PO ratio etc, and their respective amounts in the formulations.

Non-fugitive catalysts (c) being either of (c1), (c2), (c3), (c4), (c5), (c6), (c7), (c8) and (c9) type can also be made with combination of tertiary amines, e.g. by reacting an aldehyde or a ketone with more than one primary amine containing a tertiary amine group as listed under (c1).

Conversely, catalyst (c) may be made from several types of aldehyde and/or ketone groups able to react with one or more primary amines bearing tertiary amine groups.

Acid neutralization of catalyst (c) can also be considered when for instance delayed action is required. However this may be detrimental to the catalyst composition since it has to be stable when added to polyol masterbatches; ie. water, surfactant, crosslinker, etc, preferably for at least one week at room temperature. Preferably catalyst (c) is stable in a polyol premix for at least 6 months. Preferred acids are carboxylic acids, more preferably carboxylic acids with an OH group and/or a halogen moiety.

Catalysts (c) pre-reacted with polyisocyanates and polyol (b1) with no free isocyanate functions can also be used in the polyurethane formulation. Isocyanate prepolymers based on catalyst (c) can be prepared with standard equipment, using conventional methods, such a heating the catalyst (c) in a reactor and adding slowly the isocyanate under stirring and then adding eventually a polyol, or by prereacting a first polyol with a diisocyanate and then adding catalyst (c).

The isocyanates which may be used with the autocatalytic polyols of the present invention include aliphatic, cycloaliphatic, arylaliphatic and aromatic isocyanates. Aromatic isocyanates, especially aromatic polyisocyanates are preferred'.

Examples of suitable aromatic isocyanates include the 4,4'-, 2,4' and 2,2'-isomers of diphenylmethane diisocyante (MDI), blends thereof and polymeric and monomeric MDI blends toluene-2,4- and 2,6-diisocyanates (TDI), m- and p-phenylenediisocyanate, chlorophenylene-2,4-diisocyanate, diphenylene-4,4'-diisocyanate, 4,4'-diisocyanate-3,3'-dimehtyldiphenyl, 3-methyldiphenyl-methane-4,4'-diisocyanate and diphenyletherdiisocyanate and 2,4,6-triisocyanatotoluene and 2,4,4'-triisocyanatodiphenylether.

Mixtures of isocyanates may be used, such as the commercially available mixtures of 2,4- and 2,6-isomers of toluene diisocyantes. A crude polyisocyanate may also be used in the practice of this invention, such as crude toluene diisocyanate obtained by the phosgenation of a mixture of toluene diamine or the crude diphenylmethane diisocyanate obtained by the phosgenation of crude methylene diphenylamine. TDI/MDI blends may also be used. MDI or TDI based prepolymers can also be used, made either with polyol (b1), polyol (b2) or any other polyol as described heretofore. Isocyanate-terminated prepolymers are prepared by reacting an excess of polyisocyanate with polyols, including aminated polyols or imines/enamines thereof, or polyamines.

Examples of aliphatic polyisocyanates include ethylene diisocyanate, 1,6-hexamethylene diisocyanate, isophorone diisocyanate, cyclohexane 1,4-diisocyanate, 4,4'-dicyclohexylmethane diisocyanate, saturated analogues of the above mentioned aromatic isocyanates and mixtures thereof.

The preferred polyisocyantes for the production of rigid or semi-rigid foams are polymethylene polyphenylene isocyanates, the 2,2', 2,4' and 4,4' isomers of diphenylmethylene diisocyanate and mixtures thereof. For the production of flexible foams, the preferred polyisocyanates are the toluene-2,4- and 2,6-diisocyanates or MDI or combinations of TDI/MDI or prepolymers made therefrom.

Isocyanate tipped prepolymer based on non-fugitive catalyst (c) can also be used in the polyurethane formulation. It is thought that using such an autocatalytic isocyanate in a polyol isocyanate reaction mixture will reduce/eliminate the presence of unreacted isocyanate monomers. This is especially of interest with volatile isocyanates such as TDI and/or aliphatic isocyanates in coating and adhesive applications since it improves handling conditions and workers safety.

For rigid foam, the organic polyisocyanates and the isocyanate reactive compounds are reacted in such amounts that the isocyanate index, defined as the number or equivalents of NCO groups divided by the total number of isocyanate reactive hydrogen atom equivalents multiplied by 100, ranges from 80 to less than 500 preferably from 90 to 100 in the case of polyurethane foams, and from 100 to 300 in the case of combination polyurethane-polyisocyanurate foams. For flexible foams, this isocyanate index is generally between 50 and 120 and preferably between 75 and 110.

For elastomers, coating and adhesives the isocyanate index is generally between 80 and 125, preferably between 100 to 110.

For producing a polyurethane-based foam, a blowing agent is generally required. In the production of flexible polyurethane foams, water is preferred as a blowing agent. The amount of water is preferably in the range of from 0.5 to 10 parts by weight, more preferably from 2 to 7 parts by weight based on 100 parts by weight of the polyol. Carboxylic acids or salts are also used as reactive blowing agents.

In the production of rigid polyurethane foams, the blowing agent includes water, and mixtures of water with a hydrocarbon, or a fully or partially halogenated aliphatic hydrocarbon. The amount of water is preferably in the range of from 2 to 15 parts by weight, more preferably from 2 to 10 parts by weight based on 100 parts of the polyol. With an excessive amount of water, the curing rate becomes lower, the blowing process range becomes narrower, the foam density becomes lower, or the moldability becomes worse. The amount of hydrocarbon, the hydrochlorofluorocarbon, or the hydrofluorocarbon to be combined with the water is suitably selected depending on the desired density of the foam, and is preferably not more than 40 parts by weight, more preferably not more than 30 parts by weight based on 100 parts by weight of the polyol. When water is present as an additional blowing agent, it is generally present in an amount from 0.5 to 10, preferably from 0.8 to 6 and more preferably from 1 to 4 and most preferably from 1 to 3 parts by total weight of the total polyol composition.

Hydrocarbon blowing agents are volatile C₁ to C₅ hydrocarbons. The use of hydrocarbons is known in the art as disclosed in EP 421 269 and EP 695 322. Preferred hydrocarbon blowing agents are butane and isomers thereof, pentane and isomers thereof (including cyclopentane), and combinations thereof.

Examples of fluorocarbons include methyl fluoride, perfluoromethane, ethyl fluoride, 1,1-difluoroethane, 1,1,1-trifluoroethane (HFC-143a), 1,1,1,2-tetrafluoroethane (HFC-134a), 1,1,1,3,3-pentafluoropropane (HFC-245fa), 1,1,1,3,3-pentafluorobutane (HFC-365mfc), heptafluoropropane (HFC-227ea) pentafluoroethane, difluoromethane, perfluoroethane, 2,2-difluoropropane, 1,1,1-trifluoropropane, perfluoropropane, dichloropropane, difluoropropane, perfluorobutane, perfluorocyclobutane or mixtures thereof. Preferred combinations are those containing a combination of two or more of 245, 265 and 227 blowing agents..

Partially halogenated chlorocarbons and chlorofluorocarbons for use in this invention include methyl chloride, methylene chloride, ethyl chloride, 1,1,1-trichloroethane, 1,1-dichloro-1-fluoroethane (FCFC-141b), 1-chloro-1,1-difluoroethane (HCFC-142b), 1,1-dichloro-2,2,2-trifluoroethane (HCHC-123) and 1-chloro-1,2,2,2-tetrafluoroethane (HCFC-124).

Fully halogenated chlorofluorocarbons include trichloromonofluoromethane (CFC-11) dichlorodifluoromethane (CFC-12), trichlorotrifluoroethane (CFC-113), 1,1,1-trifluoroethane, pentafluoroethane, dichlorotetrafluoroethane (CFC-114), chloroheptafluoropropane, and dichlorohexafluoropropane. The halocarbon blowing agents may be used in conjunction with low-boiling hydrocarbons such as butane, pentane (including the isomers thereof), hexane, or cyclohexane or with water.

Use of carbon dioxide, either as a gas or as a liquid, as auxiliary or full blowing agent is especially of interest with the present technology. Use of artificially reduced or increased atmospheric pressure can also be applied to the present technology.

In addition to the foregoing critical components, it is often desirable to employ certain other ingredients in preparing polyurethane polymers. Among these additional ingredients are surfactants, preservatives, flame retardants, colorants, antioxidants, reinforcing agents, stabilizers and fillers.

In making polyurethane foam, it is generally preferred to employ an amount of a surfactant to stabilize the foaming reaction mixture until it cures. Such surfactants advantageously comprise a liquid or solid organosilicone surfactant. Other surfactants include polyethylene glycol ethers of long-chain alcohols, tertiary amine or alkanolamine salts of long-chain alkyl acid sulfate esters, alkyl sulfonic esters and alkyl arylsulfonic acids. Such surfactants are employed in amounts sufficient to stabilize the foaming reaction mixture against collapse and the formation of large, uneven cells. Typically, 0.2 to 3 parts of the surfactant per 100 parts by weight total polyol (b) are sufficient for this purpose

One or more catalysts for the reaction of the polyol (and water, if present) with the polyisocyanate can be used. Any suitable urethane catalyst may be used, including tertiary amine compounds, amines with isocyanate reactive groups and organometallic compounds. Preferably the reaction is carried out in the absence of a fugitive amine or an organometallic catalyst or a reduced amount as described above. Exemplary tertiary amine compounds include triethylenediamine, N-methylmorpholine, N,N-dimethylcyclohexylamine, pentamethyldiethylenetriamine, tetramethylethylenediamine, bis (dimethylaminoethyl)ether, 1-methyl-4-dimethylaminoethyl-piperazine, 3-methoxy-N-dimethylpropylamine, N-ethylmorpholine, dimethylethanolamine, N-cocomorpholine, N,N-dimethyl-N',N'-dimethyl isopropylpropylenediamine, N,N-diethyl-3-diethylaminopropylamine and dimethylbenzylamine. Exemplary organometallic catalysts include organomercury, organolead, organoferric and organotin catalysts, with organotin catalysts being preferred among these. Suitable tin catalysts include stannous chloride, tin salts of carboxylic acids such as dibutyltin di-laurate, as well as other organometallic compounds such as are disclosed in U.S. Patent 2,846,408. A catalyst for the trimerization of polyisocyanates, resulting in a polyisocyanurate, such as an alkali metal alkoxide may also optionally be employed herein. The amount of amine catalysts can vary from 0.02 to 5 percent in the formulation or organometallic catalysts from 0.001 to 1 percent in the formulation can be used. Preferably none of these catalysts are needed when non-fugitive catalyst (c) is used. A crosslinking agent or a chain extender may be added, if necessary. The crosslinking agent or the chain extender includes low-molecular polyhydric alcohols such as ethylene glycol, diethylene glycol, 1,4-butanediol, and glycerin; low-molecular amine polyol such as diethanolamine and triethanolamine; polyamines such as ethylene diamine, xlylenediamine, and methylene-bis(o-chloroaniline). The use of such crosslinking agents or chain extenders is known in the art as disclosed in U.S. Patents 4,863,979 and 4,963,399 and EP 549,120.

When preparing rigid foams for use in construction, a flame retardant is generally included as an additive. Any known liquid or solid flame retardant can be used with the autocatalytic polyols of the present invention. Generally such flame retardant agents are halogen-substituted phosphates and inorganic flame proofing agents. Common halogen-substituted phosphates are tricresyl phosphate, tris(1,3-dichloropropyl phosphate, tris(2,3-dibromopropyl) phosphate and tetrakis (2-chloroethyl)ethylene diphosphate. Inorganic flame retardants include red phosphorous, aluminum oxide hydrate, antimony trioxide, ammonium sulfate, expandable graphite, urea or melamine cyanurate or mixtures of at least two flame retardants. In general, when present, flame retardants are added at a level of from 5 to 50 parts by weight, preferable from 5 to 25 parts by weight of the flame retardant per 100 parts per weight of the total polyol present.

Fillers include, for example, barium sulfate, calcium carbonate, recycled powder foam, such as those described in EP 711,221 or in GB 922,306.

The applications for foams produced by the present invention are those known in the industry. For example rigid foams are used in the construction industry and for insulation for appliances and refrigerators. Flexible foams and elastomers find use in applications such as furniture, mattresses, shoe soles, automobile seats, sun visors, steering wheels, armrests, door panels, noise insulation parts and dashboards.

Processing for producing polyurethane products are well known in the art. In general components of the polyurethane-forming reaction mixture may be mixed together in any convenient manner, for example by using any of the mixing equipment described in the prior art for the purpose such as described in "polyurethane Handbook", by G. Oertel, Hanser publisher.

The polyurethane products are either produced continuously or discontinuously, by injection, pouring, spraying, casting, calendering, etc; these are made under free rise or molded conditions, with or without release agents, in-mold coating, or any inserts or skin put in the mold. In case of flexible foams, those can be mono- or dual-hardness.

For producing rigid foams, the known one-shot prepolymer or semi-prepolymer techniques may be used together with conventional mixing methods including impingement mixing. The rigid foam may also be produced in the form of slabstock, moldings, cavity filling, sprayed, foam, frothed foam or laminates with other material such as paper, metal, plastics or wood-board. Flexible foams are either free rise and molded while microcellular elastomers are usually molded.

The following examples are given to illustrate the invention and should not be interpreted as limiting in anyway. Unless stated otherwise, all parts and percentages are given by weight. The abbreviation mol is used for mole or moles.

A description of the raw materials used in the examples is as follows.
- DEOA: is pure diethanolamine.
- DMAPA: is 3-dimethylamino-1-propylamine.
- API: is 1-(3-aminopropyl)-imidazole a tertiary amine with a primary amine available from Aldrich.
- D.E.R.* 736 P: is an aliphatic diepoxide resin with an EEW (epoxy equivalent weight) of 190 available from The Dow Chemical Company.
- D.E.R. 732: is an aliphatic diepoxide resin with an EEW of 320 available from The Dow Chemical Company.
- D.E.R 383: is an aromatic liquid epoxy resin with an EEW of 180.4 available from The Dow Chemical Company.
- D.E.N. 438: is an aromatic liquid epoxy Novolak resin with an EEW of 190 available from The Dow Chemical Company.
- Epoxy resin A: is an aliphatic diepoxide resin with an EEW of 300 and containing less than 2 % Chlorine.
- Dabco DC 5169: is a silicone-based surfactant available from Air Products and Chemicals Inc.
- Niax Y-10184: is a silicone-based surfactant Available from G.E.
- Dabco 33 LV: is a tertiary amine catalyst available from Air Products and Chemicals Inc.
- Niax A-1: is a tertiary amine catalyst available from Crompton Corporation.
- Polyol A: is a 1,700 equivalent weight propoxylated tetrol initiated with 3,3'-diamino-N-methyl dipropylamine and capped with 15 % Ethylene oxide.
- Polyol B: is identical to polyol A but with 20 % Ethylene oxide capping.
- SPECFLEX NC 632: is a 1,700 EW polyoxypropylene polyoxyethylene polyol initiated with a blend of glycerol and sorbitol available from The Dow Chemical Company.
- Specflex NC-630: is a polyol similar to Specflex NC-632 having a lower functionality and is available from The Dow Chemical Company.
- Polyol C: is a polyol similar to Specflex NC-630 except the ethylene oxide content is increased to 17 wt%.
- SPECFLEX NC-700: is a 40 percent SAN based copolymer polyol with an average hydroxyl number of 20 available from The Dow Chemical Company.
- VORANATE T-80: is TDI 80/20 isocyanate available from The Dow Chemical Company.

All foams are made in the laboratory by preblending polyols, surfactants, crosslinkers, catalysts and water. This masterbatch is in the machine tank of a high pressure machine (Krauss-Maffei or Cannon) with' the isocyanate side filled with Voranate T-80. The reactants are poured in a 40x40x10 cm aluminum mold heated at 60°C which is subsequently closed. Prior to use the mold is sprayed with a release agent. Curing at specific demolding times is assessed by manually demolding the part and looking for defects. The minimum demolding time is reached where there is no surface defects.

Free rise tests are carried out using a 22.7 liter (5 imperial gallons) plastic bucket and pouring from the high pressure machine a shot size sufficient to fill the bucket with an approximate 30 centimeters crown of foam above the top of the bucket. Foam stability is then determined visually.

Reactivity BVT (Brookfield Viscosity Test) tests are carried out as follows: 100 grams of polyol are allowed to equilibrate at 25°C and then blended with 0.26 grams of Dabco 33 LV. Voranate T-80 is then added at a concentration corresponding to an index of 110. The viscosity build up over time is recorded until full gelation is obtained. In the case of non-fugitive catalyts (c), these are blended at various ratios with the control polyol and no Dabco 33 LV is used. Time to reach the final aimed viscosity of 20,000 mPa.s (corresponding to 100 % torque) is recorded.

### Example 1 (Comparative)

### Adduct of salicylaldehyde and 1-(3-aminopropyl)imidazole

A 100 mL two neck round bottom flask equipped with a magnetic stir bar, addition funnel, and condenser is charged with 15.0 g (0.123 mol) of salicylaldehyde. 1-3-(Aminopropyl)imidazole (15.4g, 0.123 mol) is placed in the addition funnel. The amine is added dropwise while the reaction mixture is stirred under nitrogen. After addition is complete, a bright yellow, clear oil is poured from the flask into a bottle. Isolated yield = 28.5g. Upon standing, the product solidifies and has the following properties. 1H NMR (DMSO): 8.55 (singlet, 1H), 7.65 (singlet, 1H), 7.45 (doublet, 1H), 7.3(triplet, 1H), 7.2 (singlet, 1H), 6.9 (multiplet, 3H), 4.1 (triplet, 2H), 3.5 (triplet, 2H), 3.3 (broad singlet, ~3H), 2.1 (m, 2 H); 13C NMR (DMSO-d6) 166.4, 160.5, 137.3, 132.3, 131.7, 128.5, 119.3, 118.7, 118,6, 116.4, 55.5, 43.9, 31.6. The theoretical amount of water in the product is 7.3 wt%.

### Example 2 (Comparative)

### Adduct of salicylaldehyde and 3-dimethylaminopropylamine

A 100 mL two neck round bottom flask equipped with amagnetic stir bar, addition funnel, and condenser is charged with 15.0 g (0.123 mol) of salicylaldehyde. 3-Dimethylaminopropylamine (12.55g, 0.123 mol) is placed in the addition funnel. The amine is added dropwise while the reaction mixture is stirred under nitrogen. After addition is complete, a bright yellow, clear oil is poured from the flask into a bottle. Isolated yield = 26.9g with the following properties. 1H NMR (DMSO): 8.55 (singlet, 1H), 7.45 (doublet, 1H), 7.3(triplet, 1H), 6.9 (multiplet, 2H), 3.6 (triplet, 2H), 3.4 (broad singlet, ∼3H), 2.25 (triplet, 2 H); 2.15 (singlet, 6H), 1.75 (multiplet, 2H). 13C NMR (DMSO-d6) 165.5, 160.6, 131.8, 131.2, 118.2, 118.0, 116.2, 56.2, 55.9, 44.8, 28.0. The theoretical amount of water in the product is 8.0 wt%.

### Example 3

### Adduct of Epoxy resin A, salicylaldehyde and 3-dimethylaminopropylamine

A 1L two neck round bottom flask equipped with mechanical stirrer, Claissen adapter, and gas inlet adapter connected to a vacuum / nitrogen source is charged with 444.0g (1.5 mol epoxy groups) of Epoxy resin A, 183.2 g (1.5 mol) of salicylaldehyde, and 5.8g (3.42 g active, 9.0 mmol) of tetrabutylphosphonium acetate (59wt% in methanol). The apparatus is evacuated to 20 mm Hg and then vented to nitrogen. Vacuum / nitrogen are cycled for a total of 5 times ending on nitrogen. The apparatus is left under a dynamic atmosphere of nitrogen and submerged in an oil bath held at 120°C. After 1 hour, the bath temperature is increased to 150°C and the reaction mixture is stirred over night. After 20 hours, the reaction mixture is sampled and analyzed by NMR revealing that all epoxy is consumed. The flask is removed from the oil bath and fitted with an addition funnel containing 152.3g (1.49 mol) of 3-(dimethylamino)propylamine. The amine is added dropwise to the stirred, warm reaction mixture over 1 hour. After addition is complete, a bright red, clear oil is poured from the flask into a bottle. Isolated yield = 775.2g with the following properties. 1H NMR (DMSO): 8.7 (singlet, 1H), 7.85 (doublet, 1H), 7.4 (multiplet, 1H), 7.0 (multiplet, 2H), 5.2 (broad singlet, OH), 4.0 (mulitiplet, polyether H's), 3.4 (broad muliplet, polyether + amine derived H's), 2.25 (triplet, 2H), 2.1 (singlet, 6H), 1.7 (multiplet, 2H), 1.0 (broad singlet, CH3 from polyether). The theoretical amount of water in the product is 3.4 wt%. The theoretical amount of dimethylamino groups in the sample is 1.9 meq / g.

### Example 4

### Adduct of Epoxy resin A, salicylaldehyde and 1-(3-aminopropyl)imidazole

A 1L two neck round bottom flask equipped with mechanical stirrer, Claissen adapter, and gas inlet adapter connected to vacuum / nitrogen source is charged with 450.3g (1.54 mol epoxy groups) of Epoxy resin A, 187.7 g (1.54 mol) of salicaldehyde, and 5.8g (3.42 g active, 9.0 mmol) of tetrabutylphosphonium acetate (59 wt% in methanol). The apparatus is evacuated to 20 mm Hg and then vented to nitrogen. Vacuum / nitrogen are cycled for a total of 5 times ending on nitrogen. The apparatus is left under a dynamic atmosphere of nitrogen and submerged in an oil bath held at 140°C. After 2 hours, the bath temperature is increased to 150°C and the reaction mixture is stirred over night. After 20 hours, the reaction mixture is sampled and analyzed by NMR revealing that all epoxy had been consumed. The flask is removed from the oil bath and fitted with an addition funnel containing 188.5g (1.51 mol) of 1-(3-aminopropyl)imidazole. The amine is added dropwise to the stirred, warm reaction mixture over 30 minutes. After addition is complete, an orange, clear oil is poured from the flask into a bottle. Isolated yield = 816.7g with the following properties. 1H NMR (DMSO): 8.7 (singlet, 1H), 7.85 (doublet, 1H), 7.6 (singlet, 1H), 7.4 (multiplet, 1H), 7.2 (singlet, 1H), 7.0 (multiplet, 3H), 5.2 (broad singlet, OH), 4.0 (mulitiplet, polyether + amine derived H's), 3.4 (broad muliplet, polyether + amine derived H's), 2.05 (multiplet, 2H), 1.7, 1.0 (broad singlet, CH3 from polyether). The theoretical amount of water in the product is 3.3 wt%. The theoretical amount of imidazole groups in the sample is 1.81 meq/g.

### Example 5

### Adduct of DER 732, salicylaldehyde and 3-dimethylaminopropylamine

The procedure of Example 3 is used where the reactor is charged with 450.0 g (1.4 mol epoxy groups) of DER 732 (an aliphatic liquid epoxy resin with an epoxide equivalent weight of 322), 170.7 g (1.4 mol) of salicylaldehyde, and 5.4 g (3.17 g active, 8.4 mmol) of tetrabutylphosphonium acetate. After a 20 hour reaction period, 141.8 (1.39 mol) of 3-(dimethylamino)propylamine is added dropwise over 1 hours. After addition is complete, an orange, clear oil is poured from the flask into a bottle. Isolated yield = 760.1 g having the following properties. 1H NMR (DMSO): 8.7 (singlet, 1H), 7.85 (doublet, 1H), 7.4 (multiplet, 1H), 7.0 (multiplet, 2H), 5.2 (broad multiplet, OH), 4.0 (mulitiplet, polyether H's), 3.4 (broad muliplet, polyether + amine derived H's), 2.25 (triplet, 2H), 2.1 (single, 6H), 1.7 (multiplet, 2H), 1.0 (broad singlet, CH3 from polyether). The theoretical amount of water in the product is 3.3 wt%. The theoretical amount of dimethylamino groups in the sample is 1.82 meq/g.

### Example 6

### Adduct of DER 383, salicylaldehyde and 3-dimethylaminopropylamine

To the apparatus of Example 3 is added 30.6g (169.6 mmol epoxy groups') of DER 383, 20.7 g (169.5 mmol) of salicylaldehyde, and 660.2 mg (389.5 mg active, 1.03 mmol) of tetrabutylphosphonium acetate. After a vacuum/nitrogen cycle as per Example 3, the apparatus is left under a dynamic atmosphere of nitrogen and submerged in an oil bath held at 85°C. After 2 hours, the bath temperature is increased to 100°C and the reaction mixture is stirred over night. After 20 hours, the reaction mixture is sampled and analyzed by NMR revealing that all epoxy had been consumed. The oil bath containing the reaction mixture is cooled to 70°C and the flask is fitted with an addition funnel containing 17.0g (166.4 mmol) of 3-(dimethylamino)propylamine. The amine is added dropwise to the stirred, warm reaction mixture over 10 minutes. After addition is complete, a viscous, yellow, clear oil is poured from the flask into a bottle while still warm. Isolated yield = 64 g having the following properties. 1H NMR (DMSO): 8.7 (singlet, 1H), 7.85 (doublet, 1H), 7.4 (multiplet, 1H), 7.1 (doublet 2H), 7.0 (multiplet, 2H), 6.85 (doublet, 2H), 5.5 (broad singlet, OH), 4.1 (mulitiplet, polyether H's), 3.5 (triplet, amine derived H's), 3.4 (broad singlet), 2.25 (triplet, 2H), 2.1 (singlet, 6H), 1.7 (multiplet, 2H), 1.55 (broad singlet, bisphenol A derived CH3). The theoretical amount of water in the product is 4.36 wt%. The theoretical amount of dimethylamino groups in the sample is 2.42 meq/g.

### Example 7

### Adduct of DEN 438, salicylaldehyde and 3-dimethylaminopropylamine

To the apparatus of Example 3 is added 33.6g (187.5 mmol epoxy groups) of DEN 438 (an epoxide equivalent weight of 179.2), 22.9 g (187.5 mmol) of salicylaldehyde, and 647.6 mg (382.1 mg active, 1.0 mmol) of tetrabutylphosphonium acetate. The apparatus is evacuated to 20 mm Hg and then vented to nitrogen. Vacuum / nitrogen are cycled for a total of 3 times ending on nitrogen. The apparatus is left under a dynamic atmosphere of nitrogen and submerged in an oil bath held at 90°C. After 30 minutes, the bath temperature is increased to 100°C and the reaction mixture is stirred over night. After 20 hours, the reaction mixture is sampled and analyzed by NMR revealing that all epoxy is consumed. The oil bath containing the reaction mixture is cooled to 90°C and the flask is fitted with an addition funnel containing 19.0 g (185.9 mmol) of 3-(dimethylamino)propylamine. The amine is added dropwise to the stirred, warm reaction mixture over 30 minutes. After addition is complete, a viscous, red, clear syrup is poured from the flask into a bottle while still warm. Isolated yield = 68 g. When cooled to ambient temperature the product is a clear, red glass having the following properties. 1H NMR (DMSO): 8.7 (singlet, 1H), 7.85 (doublet, 1H), 7.4 (multiplet, 1H), 6.9 (broad multiplet 5H), 5.6 (broad singlet, OH), 3-4.3 (broad mulitiplet), 2.25 (multiplet, 2H), 2.1 (broad singlet, 6H), 1.7 (multiplet, 2H). The theoretical amount of water in the product is 4.41 wt%. The theoretical amount of dimethylamino groups in the sample is 2.45 meq/g.

### Example 8

### Adduct of Epoxy resin A, vanillin, and 3-dimethylaminopropylamine

The procedure of Example 3 is used where to the apparatus is charged 30.0 g (101.4 mmol epoxy groups) of Epoxy resin A, 15.4 g (101.2 mmol) of vanillin, and 487 mg (287.3 mg active, 0.76 mmol) of tetrabutylphosphonium acetate. After an overnight reaction, 10.3 g (101.2 mmol) of 3-(dimethylamino)-propylamine is dropwise over 10 minutes. After addition was complete, a light orange/brown, clear oil is obtained. Isolated yield = 50.8g having the following properties. 1H NMR (DMSO): 8.2 (singlet, 1H), 7.35 (singlet, 1H), 7.15 (doublet, 1H, 6.95 (doublet, 1H), 5.1 (broad singlet, OH), 4.0 (mulitiplet, polyether H's), 3.8 (singlet, 3H, OCH3 derived from vanilllin), 3.4 (broad muliplet, polyether + amine derived H's), 2.25 (triplet, 2H), 2.1 (singlet, 6H), 1.7 (multiplet, 2H), 1.0 (broad singlet, CH3 from polyether). The theoretical amount of water in the product is 3.25 wt%. The theoretical amount of dimethylamino groups in the sample is 1.8 meq/g.

### Example 9

### Adduct of Epoxidized Soybean oil, vanillin, and 3-dimethylaminopropylamine

To an apparatus as per Example 3 is added 30.0 g (127.7 mmol epoxy groups) of epoxidized soybean oil (Paraplex G-62 from CP Hall Co. with an epoxide-equivalent weight of 235), 19.4 g (127.5 mmol) of vanillin, and 491.2mg (289.8 mg active, 0.76 mmol) of tetrabutylphosphonium acetate. The apparatus is evacuated to 20 mm Hg and then vented to nitrogen. This cycle is repeated 4 times and the apparatus is left under a dynamic atmosphere of nitrogen and submerged in an oil bath held at 150°C. After 30 minutes, the bath temperature is increased to 165°C and the reaction mixture stirred over night. After 14 hours, the reaction mixture is sampled and analyzed by NMR revealing that all epoxy is consumed. The oil bath containing the reaction mixture is cooled to 60°C and the flask fitted with an addition funnel containing 13.0g (127.2 mmol) of 3-(dimethylamino)propylamine. The amine is added dropwise over 10 minutes. After the addition is complete, a warm viscous syrup is obtaine. Isolated yield = 57.4g with the following analysis. 1H NMR (DMSO): 8.2 (singlet, 1H), 7.35 (multiplet, 1H), 7.0 (broad multiplet, 2H), 5.2 (broad singlet, OH), 3.2-4.6 (broad mulitiplet), 2.25 (multiplet, 2H), 2.1 (singlet, 6H), 1.7 (multiplet, 2H), 1.0-1.6 (multiplet), 0.8 (broad singlet). The theoretical amount of water in the product is 3.65 wt%. The theoretical amount of dimethylamino groups in the sample is 2.03 meq / g.

### Examples 10, 11 and 12

### Reactivity data with BVT tests

| | |
|---|---|
| Adduct | 3 parts |
| SPECFLEX NC 630 | 100 parts |
| VORANATE T-80 | index 110 |

### Example 10 (Comparative); using the Adduct of Example 2 (Comparative);

2200 cPs (2200 mPa.s) reached after 10 min.

### Example 11; using the Adduct of Example 3;

20000 cPs (20000 mPa.s) reached at 5 min 20 sec.

### Example 12; using Adduct of Example 4;

20000 cPs (20000 mPa.s) reached at 5 min 45 sec.

These data confirm that catalyst (c) catalyzes the polyol-isocyanate reaction, hence is a gelling catalyst. This is confirmed by comparative example 12C.

### Comparative example 12C

| | |
|---|---|
| Voranol NC 630 | 100 parts |
| Dabco 33 LV | 0.26 parts |
| Voranate T-80 | index 110 |

Full gelation (20,000 cPs (20,000 mPa.s)) is reached at 5 minutes 40 seconds

### Examples 23 and 14

Duplicate foaming experiments are performed using a high pressure machine equipped with a Krauss-Maffei mix-head with the adduct of example 3.

### Formulation

| | |
|---|---|
| Specflex NC-632 | 28.5 |
| Specflex NC-700 | 30 |
| Polyol A | 50 |
| Adduct example 3 | 1.5 |
| Water | 3.6 |
| DEOA | 0.7 |
| Dabco DC-5169 | 0.6 |
| Voranate T-80 | index 100 & 105 |

| Free rise foam | | Example 13 | Example 14 |
|---|---|---|---|
| | Cream time (s) | 4 | 4 |
| | Gel time (s) | 61 | 60 |
| | Rise time (s) | 131 | 133 |
| | Free rise density (kg/m3) | 28 | NA |

Molded foam: demolding time 4', molded density 38.4 kg/m3. Examples 13 and 14 show that good, stable foams are obtained when catalyst (c) is combined with a polyol having catalytic activity (polyol A) and conventional polyols. No other catalysts are used with examples 13 and 14. No amine odors are detected at demold.

### Examples 15, 16

For example 15, the Adduct of Example 4 is used in place of the Adduct of Example 3, with the formulation of Examples 13/14; index 100: Reactivity measured: Cream Time 5 s; Gel Time 70 s; Rise Time 157 s. A good foam is obtained with a free Rise Density of 28.5 kg/m3.

For example 16, the Adduct of Example 5 is used in place of the Adduct of Example 3, with the formulation of examples 13/14. Index 100: Reactivity measured: Cream Time 4 s; Gel Time 58 s; Rise Time 126 s. A Good foam is obtained with a free Rise Density 28 kg/m3.

### Examples 17 and 18

Foaming tests are done using a Cannon machine

For Example 17 the adduct of Example 3 is used in the following formulation.

### Formulation

| | |
|---|---|
| Polyol C | 24.4 |
| Specflex NC-700 Polyol | 37.5 |
| Polyol B | 36.6 |
| Adduct example 3 | 1.5 |
| Water | 3.9 |
| DEOA | 1.4 |
| Niax Y-10184 | 1.2 |
| VORANATE T-80 | index 105 |

Size: 600 grams in 5 gallon (20 Litre) bucket. Reactivity measured: Cream Time 5 s; Rise Time 84 s. Good foam obtained with free Rise Density 28.8 kg/m3

For Example 18 the adduct of Example 5 is used with the formulation of Example 17.

Size: 600 grams in 5 gallon (20 Litre) bucket. Reactivity measured: Cream Time 5 s; Rise Time 83 s. Good foam obtained with free Rise Density 28.4 kg/m3

### Example 19

The adduct of Example 3 is blended with polyol B and C at various levels and an aging study is carried out by measuring reactivity through the BVT test and by visual inspection to record any sign of phase separation. After 13 weeks at 60°C no loss of reactivity nor sign of phase separation is observed with the following blend:

| | |
|---|---|
| Adduct example 3 | 5 parts by weight |
| Polyol B | 38 |
| Polyol C | 57 |

### Example 20

A polyol blend is prepared with the following composition by weight:

| | |
|---|---|
| Specflec NC-632 | 18.5 |
| Specflex NC-700 | 30 |
| Polyol A | 50 |
| Adduct example 5 | 1.5 |
| Water | 3.6 |
| DEOA | 0.7 |
| Dabco DC-5169 | 0.6 |

This blend is foamed with Voranate T-80 using a Krauss-Maffei mix-head at various days:

| | Day 1 | Day 4 |
|---|---|---|
| | | |
| Cream time (s) | 5 | 5 |
| Gel time (s) | 69 | 70 |
| Rise time (s) | 141 | 143 |
| Free Rise density (kg/m3) | 30 | 29 |

These aging data show that the polyol blend containing water and imine based catalyst (c) is stable over several days.

### Example 21

### Adduct of DER 732, salicylaldehyde, 1-(3-aminopropyl)imidazole, and 3-dimethylaminopropylamine

The procedure of Example 3 is followed where the apparatus is charged with 475.0g (1.498 mol epoxy groups) of DER 732, 173.8 g (1.424 mol) of salicylaldehyde, and 5.8g (3.42 g active, 9.0 mmol) of tetrabutylphosphonium acetate. The reaction is allowed to proceed for 16 hours after which time 72.7g (0.712 mol) of 3-(dimethylamino)propylamine and 89.1g (0.712 mol) of 1-(3-aminopropyl)imidazole is added dropwise via an addition funnel. The amine is added dropwise to the stirred, warm reaction mixture over 1 hour. After addition is complete, an orange, clear oil is obtained. Isolated yield = 805.9g. The theoretical amount of water in the product is 3.15 wt%. The theoretical amount of total amine functionality in the sample is 1.75 meq /g divided equally between dimethylamino groups and imidazole groups.

### Example 22

Foaming is done with 1.5 parts by weight of adduct of Example 21 using formulation and conditions of Examples 14 and 15:

| | |
|---|---|
| Cream time (s) | 4 |
| Gel time (s) | 69 |
| Rise time (s) | 129 |

This formulation is used to mold foam parts at molded densities of 38 kg/m3 with good curing at 4 minutes demolding time.

### Example 23

### Adduct of Epoxy resin A, salicylaldehyde, bis-phenol A and 3-dimethylaminopropylamine

A 1 L neck round bottom flask equipped with mechanical stirrer, Claissen adapter, and gas inlet adapter connected to vacuum/nitrogen source is charged with 500.0 g (1.69 mol epoxy groups) of Epoxy resin A, 103. g (0.845 mol) of salicylaldehyde, 96.45 g (0.4435 mol) of bisphenol A, and 6.5 g of tetrabutylphosphonium acetate (59 % in methanol). The apparatus is evacuated to 20 mm Hg and then vented to nitrogen. Vacuum/nitrogen are cycled for a total of 5 times ending on nitrogen. The apparatus is left under a dynamic atmosphere of nitrogen and submerged in an oil bath held at 120° C. After 1 hour, the bath temperature is increased to 150°C and the reaction mixture is stirred over night. After 20 hours, the reaction mixture is sampled and analyzed by NMR revealing that all epoxy is consumed. The flask is removed from the oil bath and fitted with an addition funnel containing 86.4 (0.845 mol) of 3-(dimethylamino)propylamine. The amine is added dropwise to the stirred, warm reaction mixture over 1 hour. After addition is complete, the orange, clear oil is poured from the flask into a bottle. Isolated yield = 780.4 g.

### Example 24

### Adduct of Epoxy resin A, 3,3'diamino-N-methyl-dipropylamine, and 3-dimethylaminopropylamine

The procedure of Example 23 is followed using 444 g (1.5 mol epoxy group) of Epoxy resin A, 183.2 g (1.5 mol) of salicylaldehyde, and 5.8 g of tetrabutylphosphonium acetate (59 % weight in methanol). After reaction overnight at 150°C a mixture of 76.6 g (0.75 mol) of 3-dimethylaminopropylamine and 54.5 g (0.375 mol) of 3,3'-diamino-N-methyldipropylamine is added to the reactants. Isolated yield = 752.9 g

Other embodiments of the invention will be apparent to those skilled in the art from a consideration of this specification or practice of the invention disclosed herein. It is intended that the specification and examples be considered as exemplary only, with the true scope and spirit of the invention being indicated by the following claims.

## Claims

1. A catalyst composition wherein the catalyst has at least one imine linkage and at least one tertiary amine moiety wherein the imine linkage is obtained by the reaction mixture comprising
(i) a compound having at least one aldehyde or ketone moiety and
(ii) a compound having at least one primary amine moiety and at least one tertiary amine moiety
wherein (i) is mixture of (a) a compound containing at least one epoxy moiety with (b) a compound containing an epoxy reactive moiety and an aldehyde or ketone.

2. The catalyst of Claim 1 wherein the compound having both primary and tertiary amine moities is represented by the formula:
H₂N - R⁸ - N(R⁹)₂ where R⁸ is an aliphatic or cyclic chain having 1 to 20 carbon atoms and R⁹ is a C1 to C3 alkyl group.

3. The catalyst of Claim 1 wherein the compound having both primary and tertiary amine moieties is 3-(dimethylamino)-propylamine, 1-(3-aminopropyl)-imidazole, 1-(3-aminopropyl)-2-methylimidazole, N,N-dimethyldipropylenetriamine, N,N-dimethylethylene diamine, N,N-diethylethylene diamine, N,N-dibutylethylene diamine, 3-(diethylamino)-propylamine, 3-(dibutylamino)-propylamine, N,N,2,2-tetramethyl-1,3-propanediamine, 2-amino-5-diethylaminopentane, N-methyl- (N'-aminoethyl)-piperazine, 1,4-bis(3-aminopropyl)piperazine, 3-aminoquinuclidine, 4-(2-aminoethyl)morpholine, 4-(3-aminopropyl)morpholine, N,N-dimethyl-1,4-phenylenediamine, 5-amino-1-ethylpyrazole, 2-aminopyridine, 2-(aminomethyl)pyridine, 2-(aminoethyl)pyridine, 4-aminopyridine, 3-aminopyridine, 3-(aminomethyl)pyridine, N-aminopropyl pyrrolidine, 2-aminopicolines, diaminopyridines, 2-aminopyrimidine, 4-aminopyrimidine, aminopyrazine, 3-amino-1,2,4-triazine, aminoquinolines, N,N dimethyldipropylenetriamine and 3,3'-diamino-N-methyl dipropylamine, N-methyl-1,3-propyldiamine

4. The catalyst of Claim 1 wherein the epoxy reactive moiety is an alcohol, amine, thiol or carboxylic acid.

5. The catalyst of Claim 1 wherein the compound having an aldehyde moiety and an epoxide reactive moiety is a C3 to C30 aliphatic, aromatic or polyaromatic compound or a ring structure containing a heteroatom, with the proviso when the compound having an aldehyde and epoxide moiety contains a ring structure, the aldehyde moiety is bonded directly to the ring and the epoxide reactive moiety is bonded directly to the ring or bonded to the ring via a C3 to C6 linear or branched alkyl.

6. The catalyst of Claim 5 wherein the compound having an epoxide reactive moiety and an aldehyde moiety is salicylaldehyde, vanillin, 5-(hydroxymethyl)-furfural, 3-hydroxybenzaldehyde, 4-hydroxybenzaldehyde, dihydroxybenzaldehydes, trihydroxybenzaldehydes, 2-carboxybenzaldehyde, 3-carboxybenzaldehyde or a mixture thereof.

7. The catalyst of Claim 1 wherein the compound having a ketone and an epoxide functional moiety is a C3 to C30 aliphatic, aromatic or polyaromatic compound or a ring structure containing a heteroatom with the proviso when the compound having a ketone and epoxide moieties contains a ring structure, the epoxide reactive moiety is bonded directly to the ring or bonded via a C1 to C6 linear or branched alkyl.

8. The catalyst of Claim 4 wherein the compound having a ketone and epoxide functionality is 2'-hydroxyacetophenone, 4'-hydroxyacetophenone, 3'-hydroxyacetophenone, 3-acetyl-1-propanol, 4-hydroxy-3-methyl-2-butanone, 4-hydroxy-4-methyl-2-pentanone, 4'-hydroxyvalerophenone, dihydroxyacetophenone, benzyl-4-hydroxyphenylketone, acetovanillone, 3'-aminoacetophenone, 4'-aminoacetophenone, aminobenzophenone, 4-acetylbenzoic acid, 2-benzoylbenzoic acid or a mixture thereof.

9. The catalyst of Claim 1 wherein the compound containing at least one epoxide moiety is represented by the formula: 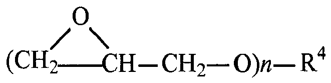 wherein R⁴ is substituted or unsubstituted aromatic, aliphatic, cycloaliphatic or heterocyclic group and n has an average value of from 1 to 8.

10. The catalyst of Claim 1 wherein in step (i) the mixture further contains a phenol, cresol, bis phenol A, bisphenol F, a novolak polyol, ethylenediamine, 3,3'-diamino-N-methyl-dipropylamine, resorcinol, adipic acid, succinic acid, isophthalic acid, phthalic acid, terephthalic acid, acetic acid, or a combination thereof.

11. The catalyst of Claim 1 wherein in step (ii) the compound containing a primary amine and a tertiary amine moieties contains two or more primary amines.

12. The catalyst of Claim 1 wherein 1 to 50 percent of the epoxy moieties present in step (a) are reacted with a compound containing an epoxy reactive group and a tertiary amine moiety.

13. A polyol composition containing from 99.9 to 50 percent by weight of a polyol compound or blend of polyols having a functionality of 2 to 8 and a hydroxyl number of from 20 to 800 and from 0.1 to 50 percent of a catalyst composition according to claim 1.

14. A process for the production of a polyurethane product by reaction of a mixture of
(a) at least one organic polyisocyanate with
(b) a polyol composition wherein the polyol has a calculated nominal functionality between 2 to 8 and a hydroxyl number of from 20 to 800 and
(c) at least one non-fugitive catalyst according to any one of claims 1 to 12
(d) optionally in the presence of another catalyst and/or blowing agent; and
(e) optionally additives or auxiliary agents known per se for the production of polyurethane foams, elastomers or coatings.

15. The process of Claim 14 wherein the catalyst is present in an amount from 0.1 to 50 weight percent of the total weight of (b) and (c).

16. The process of Claim 14 for producing a flexible polyurethane foam wherein the polyol composition has a hydroxyl number from 20 to 100 and the blowing agent is water in an amount of 0.2 to 10 weight percent of the polyol.

17. The process of Claim 14 for producing a rigid polyurethane foam where the polyol composition has an average hydroxyl number from 200 to 1000 and the blowing agent is water in combination with a hydrocarbon or a hydrofluorocarbon.

## Patentansprüche

1. Eine Katalysatorzusammensetzung, wobei der Katalysator mindestens eine Iminbindung und mindestens einen tertiären Aminteil aufweist, wobei die Iminbindung durch die Reaktionsmischung erhalten wird, die Folgendes beinhaltet:
(i) eine Verbindung, die mindestens einen Aldehyd- oder Ketonteil aufweist, und
(ii) eine Verbindung, die mindestens einen primären Aminteil und mindestens einen tertiären Aminteil aufweist,
wobei (i) eine Mischung ist von (A) einer Verbindung, die mindestens einen Epoxidteil enthält, mit (B) einer Verbindung, die einen epoxidreaktiven Teil und ein Aldehyd oder Keton enthält.

2. Katalysator gemäß Anspruch 1, wobei die Verbindung, die sowohl einen primären als auch einen tertiären Aminteil aufweist, durch die folgende Formel dargestellt wird:
H₂N - R⁸ - N(R⁹)₂, wobei R⁸ eine aliphatische oder zyklische Kette ist, die 1 bis 20 Kohlenstoffatome aufweist, und R⁹ eine C1- bis C3-Alkylgruppe ist.

3. Katalysator gemäß Anspruch 1, wobei die Verbindung, die sowohl einen primären als auch einen tertiären Aminteil aufweist, 3-(Dimethylamino)-propylamin, 1-(3-Aminopropyl)-imidazol, 1-(3-Aminopropyl)-2-methylimidazol, N,N-Dimethyldipropylentriamin, N,N-Dimethylethylendiamin, N,N-Diethylethylendiamin, N,N-Dibutylethylendiamin, 3-(Diethylamino)-propylamin, 3-(Dibutylamino)-propylamin, N,N,2,2-Tetramethyl-1,3-propandiamin, 2-Amino-5-diethylaminopentan, N-Methyl-(N'-aminoethyl)-piperazin, 1,4-Bis(3-aminopropyl)piperazin, 3-Aminochinuclidin, 4-(2-Aminoethyl)morpholin, 4-(3-Aminopropyl)morpholin, N,N-Dimethyl-1,4-phenylendiamin, 5-Amino-1-ethylpyrazol, 2-Aminopyridin, 2-(Aminomethyl)pyridin, 2-(Aminoethyl)pyridin, 4-Aminopyridin, 3-Aminopyridin, 3-(Aminomethyl)pyridin, N-Aminopropylpyrrolidin, 2-Aminopicoline, Diaminopyridine, 2-Aminopyrimidin, 4-Aminopyrimidin, Aminopyrazin, 3-Amino-1,2,4-triazin, Aminochinoline, N,N-Dimethyldipropylentriamin und 3,3'-Diamino-N-methyldipropylamin, N-Methyl-1,3-propyldiamin ist.

4. Katalysator gemäß Anspruch 1, wobei der epoxidreaktive Teil ein Alkohol, ein Amin, ein Thiol oder eine Carbonsäure ist.

5. Katalysator gemäß Anspruch 1, wobei die Verbindung, die einen Aldehydteil und einen epoxidreaktiven Teil aufweist, eine aliphatische, aromatische oder polyaromatische C3-bis C30-Verbindung ist oder eine Ringstruktur ist, die ein Heteroatom enthält, mit der Maßgabe, dass, wenn die Verbindung, die einen Aldehyd- und einen Epoxidteil aufweist, eine Ringstruktur enthält, der Aldehydteil direkt an den Ring gebunden ist und der epoxidreaktive Teil direkt an den Ring gebunden ist oder über ein lineares oder verzweigtes C3- bis C6-Alkyl an den Ring gebunden ist.

6. Katalysator gemäß Anspruch 5, wobei die Verbindung, die einen epoxidreaktiven Teil und einen Aldehydteil aufweist, Salicylaldehyd, Vanillin, 5-(Hydroxymethyl)-furfural, 3-Hydroxybenzaldehyd, 4-Hydroxybenzaldehyd, Dihydroxybenzaldehyde, Trihydroxybenzaldehyde, 2-Carboxybenzaldehyd, 3-Carboxybenzaldehyd oder eine Mischung davon ist.

7. Katalysator gemäß Anspruch 1, wobei die Verbindung, die einen Keton- und einen epoxidfunktionellen Teil aufweist, eine aliphatische, aromatische oder polyaromatische C3- bis C30-Verbindung ist oder eine Ringstruktur ist, die ein Heteroatom enthält, mit der Maßgabe, dass, wenn die Verbindung, die einen Keton- und einen Epoxidteil aufweist, eine Ringstruktur enthält, der epoxidreaktive Teil direkt an den Ring gebunden ist oder über ein lineares oder verzweigtes C1- bis C6-Alkyl gebunden ist.

8. Katalysator gemäß Anspruch 4, wobei die Verbindung, die eine Keton- und Epoxidfunktionalität aufweist, 2'-Hydroxyacetophenon, 4'-Hydroxyacetophenon, 3'-Hydroxyacetophenon, 3-Acetyl-1-propanol, 4-Hydroxy-3-methyl-2-butanon, 4-Hydroxy-4-methyl-2-pentanon, 4'-Hydroxyvalerophenon, Dihydroxyacetophenon, Benzyl-4-hydroxyphenylketon, Acetovanillon, 3'-Aminoacetophenon, 4'-Aminoacetophenon, Aminobenzophenon, 4-Acetylbenzoesäure, 2-Benzoylbenzoesäure oder eine Mischung davon ist.

9. Katalysator gemäß Anspruch 1, wobei die Verbindung, die mindestens einen Epoxidteil enthält, durch die folgende Formel dargestellt wird: 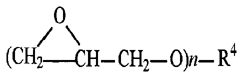 wobei R⁴ eine substituierte oder nicht substituierte aromatische, aliphatische, cycloaliphatische oder heterocyclische Gruppe ist und n einen durchschnittlichen Wert von 1 bis 8 aufweist.

10. Katalysator gemäß Anspruch 1, wobei die Mischung in Schritt (i) ferner ein Phenol, Kresol, Bisphenol A, Bisphenol F, ein Novolakpolyol, Ethylendiamin, 3,3'-Diamino-N-methyl-dipropylamin, Resorcinol, Adipinsäure, Bernsteinsäure, Isophthalsäure, Phthalsäure, Terephthalsäure, Essigsäure oder eine Kombination davon enthält.

11. Katalysator gemäß Anspruch 1, wobei die Verbindung, die einen primären Amin- und einen tertiären Aminteil enthält, in Schritt (ii) zwei oder mehr primäre Amine enthält.

12. Katalysator gemäß Anspruch 1, wobei 1 bis 50 Prozent der in Schritt (A) vorhandenen Epoxidteile mit einer Verbindung zur Reaktion gebracht werden, die eine epoxidreaktive Gruppe und einen tertiären Aminteil enthält.

13. Eine Polyolzusammensetzung, die zu von 99,9 bis 50 Gewichtsprozent eine Polyolverbindung oder ein Gemisch von Polyolen mit einer Funktionalität von 2 bis 8 und einer Hydroxylzahl von 20 bis 800 und zu von 0,1 bis 50 Prozent eine Katalysatorzusammensetzung gemäß Anspruch 1 enthält.

14. Ein Verfahren zur Produktion eines Polyurethanprodukts durch die Reaktion einer Mischung von
(a) mindestens einem organischen Polyisocyanat mit
(b) einer Polyolzusammensetzung, wobei das Polyol eine berechnete nominale Funktionalität von zwischen 2 bis 8 und eine Hydroxylzahl von 20 bis 800 aufweist, und
(c) mindestens einem nichtflüchtigen Katalysator gemäß einem der Ansprüche 1 bis 12,
(d) wahlweise in Gegenwart eines anderen Katalysators und/oder Treibmittels; und
(e) wahlweise an sich bekannten Zusätzen oder Hilfsstoffen für die Produktion von Polyurethanschäumen, Elastomeren oder Beschichtungen.

15. Verfahren gemäß Anspruch 14, wobei der Katalysator in einer Menge von 0,1 bis 50 Gewichtsprozent des Gesamtgewichts von (b) und (c) vorhanden ist.

16. Verfahren gemäß Anspruch 14 zum Produzieren eines weichen Polyurethanschaums, wobei die Polyolzusammensetzung eine Hydroxylzahl von 20 bis 100 aufweist und das Treibmittel Wasser in einer Menge von 0,2 bis 10 Gewichtsprozent des Polyols ist.

17. Verfahren gemäß Anspruch 14 zum Produzieren eines festen Polyurethanschaums, wobei die Polyolzusammensetzung eine durchschnittliche Hydroxylzahl von 200 bis 1000 aufweist und das Treibmittel Wasser in Kombination mit einem Kohlenwasserstoff oder einen Fluorkohlenwasserstoff ist.

## Revendications

1. Une composition de catalyseur dans laquelle le catalyseur a au moins une liaison imine et au moins un groupement amine tertiaire, la liaison imine étant obtenue par le mélange réactionnel comprenant
(i) un composé ayant au moins un groupement aldéhyde ou cétone et
(ii) un composé ayant au moins un groupement amine primaire et au moins un groupement amine tertiaire
dans laquelle (i) est un mélange (A) d'un composé contenant au moins un groupement époxy avec (B) un composé contenant un groupement réactif époxy et un aldéhyde ou une cétone.

2. Le catalyseur de la revendication 1 dans lequel le composé ayant à la fois des groupements amine primaire et amine tertiaire est représenté par la formule :
H₂N - R⁸ - N(R⁹)₂ où R⁸ est une chaîne aliphatique ou cyclique ayant de 1 à 20 atomes de carbone et R⁹ est un groupe alkyle en C1 à C3.

3. Le catalyseur de la revendication 1 dans lequel le composé ayant à la fois des groupements amine primaire et amine tertiaire est la 3-(diméthylamino)-propylamine, le 1-(3-aminopropyl)-imidazole, le 1-(3-aminopropyl)-2-méthylimidazole, la N,N-diméthyldipropylènetriamine, la N,N-diméthyléthylène diamine, la N,N-diéthyléthylène diamine, la N,N-dibutyléthylène diamine, la 3-(diéthylamino)-propylamine, la 3-(dibutylamino)-propylamine, la N,N,2,2-tétraméthyl-1,3-propanediamine, le 2-amino-5-diéthylaminopentane, la N-méthyl-(N'-aminoéthyl)-pipérazine, la 1,4-bis(3-aminopropyl)pipérazine, la 3-aminoquinuclidine, la 4-(2- aminoéthyl)morpholine, la 4-(3-aminopropyl)morpholine, la N,N-diméthyl-1,4-phénylène diamine, le 5-amino-1-éthylpyrazole, la 2-aminopyridine, la 2-(aminométhyl)pyridine, la 2-(aminoéthyl)pyridine, la 4-aminopyridine, la 3-aminopyridine, la 3-(aminométhyl)pyridine, des N-aminopropyl pyrrolidine, 2-aminopicolines, des diaminopyridines, la 2-aminopyrimidine, la 4-aminopyrimidine, l'aminopyrazine, la 3-amino-1,2,4-triazine, des aminoquinolines, la N,N-diméthyldipropylène triamine et la 3,3'-diamino-N-méthyl dipropylamine, la N-méthyl-1,3-propyldiamine.

4. Le catalyseur de la revendication 1 dans lequel le groupement réactif époxy est un alcool, une amine, un thiol ou un acide carboxylique.

5. Le catalyseur de la revendication 1 dans lequel le composé ayant un groupement aldéhyde et un groupement réactif époxyde est un composé aliphatique, aromatique ou polyaromatique en C3 à C30 ou une structure de cycle contenant un hétéroatome, à la condition que lorsque le composé ayant un groupement aldéhyde et un groupement époxyde contient une structure de cycle, le groupement aldéhyde est directement lié au cycle et le groupement réactif époxyde est directement lié au cycle ou lié au cycle par l'intermédiaire d'un alkyle linéaire ou ramifié en C3 à C6.

6. Le catalyseur de la revendication 5 dans lequel le composé ayant un groupement réactif époxyde et un groupement aldéhyde est l'aldéhyde salicylique, la vanilline, le 5-(hydroxyméthyl)-furfural, le 3-hydroxybenzaldéhyde, le 4-hydroxybenzaldéhyde, des dihydroxybenzaldéhydes, des trihydroxybenzaldéhydes, le 2-carboxybenzaldéhyde, le 3-carboxybenzaldéhyde ou un mélange de ceux-ci.

7. Le catalyseur de la revendication 1 dans lequel le composé ayant un groupement cétone et un groupement fonctionnel époxyde est un composé aliphatique, aromatique ou polyaromatique en C3 à C30 ou une structure de cycle contenant un hétéroatome, à la condition que lorsque le composé ayant un groupement cétone et un groupement époxyde contient une structure de cycle, le groupement réactif époxyde est directement lié au cycle ou lié par l'intermédiaire d'un alkyle linéaire ou ramifié en C1 à C6.

8. Le catalyseur de la revendication 4 dans lequel le composé ayant une fonctionnalité cétone et époxyde est la 2'-hydroxyacétophénone, la 4'-hydroxyacétophénone, la 3'-hydroxyacétophénone, le 3-acétyl-1-propanol, la 4-hydroxy-3-méthyl-2-butanone, la 4-hydroxy-4-méthyl-2-pentanone, la 4'-hydroxyvalérophénone, la dihydroxyacétophénone, la benzyl-4-hydroxyphénylcétone, l'acétovanillone, la 3'-aminoacétophénone, la 4'-aminoacétophénone, l'aminobenzophénone, l'acide 4-acétylbenzoïque, l'acide 2-benzoylbenzoïque ou un mélange de ceux-ci.

9. Le catalyseur de la revendication 1 dans lequel le composé contenant au moins un groupement époxyde est représenté par la formule : 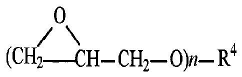 dans laquelle R⁴ est un groupe aromatique, aliphatique, cycloaliphatique ou hétérocyclique substitué ou non substitué et n a une valeur moyenne allant de 1 à 8.

10. Le catalyseur de la revendication 1 dans lequel dans l'étape (i) le mélange contient en outre un phénol, du crésol, du bisphénol A, du bisphénol F, un polyol novalaque, de l'éthylène diamine, de la 3,3'-diamino-N-méthyl-dipropylamine, du résorcinol, de l'acide adipique, de l'acide succinique, de l'acide isophtalique, de l'acide phtalique, de l'acide téréphtalique, de l'acide acétique, ou une combinaison de ceux-ci.

11. Le catalyseur de la revendication 1 dans lequel dans l'étape (ii) le composé contenant un groupement amine primaire et un groupement amine tertiaire contient deux amines primaires ou plus.

12. Le catalyseur de la revendication 1 dans lequel de 1 à 50 pour cent des groupements époxy présents dans l'étape (A) sont amenés à réagir avec un composé contenant un groupe réactif époxy et un groupement amine tertiaire.

13. Une composition de polyol contenant de 99,9 à 50 pour cent en poids d'un composé polyol ou d'un mélange homogène de polyols ayant une fonctionnalité allant de 2 à 8 et un indice d'hydroxyle allant de 20 à 800 et de 0,1 à 50 pour cent d'une composition de catalyseur selon la revendication 1.

14. Un procédé destiné à la production d'un produit de polyuréthane par la réaction d'un mélange de
(a) au moins un polyisocyanate organique avec
(b) une composition de polyol, le polyol ayant une fonctionnalité nominale calculée comprise entre 2 et 8 et un indice d'hydroxyle allant de 20 à 800 et
(c) au moins un catalyseur non fugitif selon l'une quelconque des revendications 1 à 12
(d) facultativement en présence d'un autre catalyseur et/ou agent d'expansion ; et
(e) facultativement des additifs ou des agents auxiliaires connus en soi destinés à la production de mousses, d'élastomères ou d'enduits de polyuréthane.

15. Le procédé de la revendication 14 dans lequel le catalyseur est présent dans une quantité allant de 0,1 à 50 pour cent en poids du poids total de (b) et (c).

16. Le procédé de la revendication 14 destiné à produire une mousse de polyuréthane souple dans lequel la composition de polyol a un indice d'hydroxyle allant de 20 à 100 et l'agent d'expansion est de l'eau dans une quantité allant de 0,2 à 10 pour cent en poids du polyol.

17. Le procédé de la revendication 14 destiné à produire une mousse de polyuréthane rigide où la composition de polyol a un indice d'hydroxyle moyen allant de 200 à 1 000 et l'agent d'expansion est de l'eau en combinaison avec un hydrocarbure ou un hydrofluorocarbure.
